# EUROPEAN PATENT APPLICATION

(11) **EP 4 674 382 A1**
(43) Date of publication of application: **07.01.2026**
(21) Application number: 24777591.9
(22) Date of filing: 27.02.2024
(51) Int. Cl.: A61F 2/24, A61B 17/00

(54) **INTERVENTIONAL HANDLE AND INTERVENTIONAL DELIVERY SYSTEM**

(30) Priority: 31.03.2023 CN 202310339819; 31.03.2023 CN 202310339770
(71) Applicant: Shanghai MicroPort CardioFlow Medtech Co., Ltd., Shanghai 201203 (CN)
(72) Inventor: LIN, Xing, Shanghai 201203 (CN); LU, Shigui, Shanghai 201203 (CN); HUANG, Qingqing, Shanghai 201203 (CN); CHEN, Guoming, Shanghai 201203 (CN)
(74) Representative: Patentship Patentanwaltsgesellschaft mbH
(86) International application number: PCT/CN2024/078668
(87) International publication number: WO 2024/198801

(57) **Abstract**

The present invention provides an interventional handle and interventional delivery system. The interventional handle includes a housing, an electric drive module, a manual drive module, a cover and a control module. The housing defines an opening, and the cover is complementary in shape to the opening and adapted to detachably cover and close the opening. The electric drive module is disposed inside the housing, and the manual drive module is arranged within the housing in positional correspondence with the opening. The control module is configured to: obtain assembly information indicating whether the cover is covering and closing the opening; and if the assembly information indicates that the cover is covering and closing the opening, switch the electric drive module to an enabled mode, or if the assembly information indicates that the cover is separate from the opening, switch the electric drive module to a disabled mode. This optimized interventional handle layout allows for the incorporation of more other functional modules, and the cover may be separated from the housing in order to address a need for manual actuation.

## Description

### TECHNICAL FIELD

The present invention relates to the field of medical devices, and more particularly to interventional handles and interventional delivery systems.

### BACKGROUND

Interventional therapy is a brand new therapeutic technique, which has emerged abroad in recent years as a minimally invasive alternative therapeutic option based on modern high technologies. It involves introducing, into a patient's body under the guidance of medical imaging equipment, a specialized precision instrument for diagnosis and local treatment of a lesion therein. This technique features absence of considerable incisions, minor trauma, fast recovery and good outcomes, and avoids possible harm of traditional surgery to patients.

Delivery systems used in interventional procedures for heart valve treatment are generally composed of interventional catheters and an interventional handle. Serving as a power source for the entire procedure, the interventional handle is required to provide sufficient safety and effectiveness. To date, conventional purely manual interventional handles rely on threaded fits for power transmission, while electric interventional handles employ a combination of components such as a motor, a shaft coupling, gears and a screw or hydraulic component. As the research on the treatment of cardiac valve disease goes deeper, hybrid power interventional handles with higher release accuracy and stability have been developed for this purpose, such as electric/manual and hydraulic/manual interventional handles. These hybrid power interventional handles operate mainly in an electric or hydraulic mode, with a manual mode of operation being reserved as a risk management measure. When the main mode of operation fails, the system can be switched to the manual mode to complete the procedure, reducing risks associated with such procedures. In view of the manual mode, which is indispensable but used at an extremely low frequency, it is necessary to further increase structural harmoniousness and naturalness of the hybrid power interventional handles by sensibly incorporating the manual functionality in the main structure so as to have no advance impact on the latter while playing its role in the case of an emergency.

### SUMMARY

It is an object of the present invention to provide interventional handles and interventional delivery systems, which overcome the problems with existing delivery systems arising from a suboptimal overall interventional handle layout due to an indispensable manual module.

To this end, the present invention provides an interventional handle, which includes a housing, an electric drive module, a manual drive module, a cover and a control module.

The housing defines an opening, and the cover is complementary in shape to the opening and adapted to detachably cover and close the opening. The electric drive module is disposed inside the housing, and the manual drive module is arranged within the housing in positional correspondence with the opening.

The control module is configured to: obtain assembly information indicating whether the cover is covering and closing the opening; and if the assembly information indicates that the cover is covering and closing the opening, switch the electric drive module to an enabled mode, or if the assembly information indicates that the cover is separate from the opening, switch the electric drive module to a disabled mode.

Optionally, the interventional handle may include a first sensor disposed within the housing in positional correspondence with the opening, which is connected to the control module and adapted to obtain the assembly information indicating whether the cover is covering and closing the opening.

Optionally, the first sensor may be a magnetic sensor, wherein the cover is provided with a magnet in positional correspondence with the magnetic sensor.

Optionally, the interventional handle may include mating connector members adapted to applying a force for urging the cover toward the opening and assembling the cover at the opening.

Optionally, the connector members may include a first magnetic attraction member and a second magnetic attraction member, which can attract and mate with each other, the first magnetic attraction member disposed on the cover, the second magnetic attraction member disposed at the opening, wherein when the cover is covering and closing the opening, the cover is attractively retained at the opening by a magnetic force.

Optionally, the cover may define an engagement member and the housing may define a mating engagement member, wherein when the cover is covering and closing the opening, the cover is engaged with the opening by the engagement members, and wherein each of the engagement members and a respective one of the connector members are spaced apart from each other along an axis of the housing at opposite ends of the cover or opening.

Optionally, the interventional handle may include a conversion module including a first screw arranged along an axis of the housing and a ball nut in rotatable, threaded engagement with the first screw, the first screw adapted for connection with an outer tube in an interventional catheter,
wherein the housing restricts the ball nut in position with respect to the axis of the housing and restricts circumferential rotation of the first screw, and the ball nut is connected to both the electric and manual drive modules and is adapted to be rotated around the first screw by the electric or manual drive module to drive the first screw to move along the axis of the housing.

Optionally, the manual drive module may include a first knob connected coaxially to the ball nut, wherein the electric drive module includes a motor and a power transmission member, the motor connected to the ball nut via the power transmission member.

Optionally, the interventional handle may include a second sensor and a third sensor, the second sensor adapted to obtain information about a distal travel limit for the first screw, the third sensor adapted to obtain information about a proximal travel limit for the first screw, wherein the control module prevents the electric drive module from causing further distal movement of the first screw based on the information about the distal travel limit and prevents the electric drive module from causing further proximal movement of the first screw based on the information about the proximal travel limit.

Optionally, the second and third sensors may be magnetic sensors, and the first screw may be provided with a magnet, wherein upon the first screw reaching the distal travel limit during its travel along axis of the housing, the magnet comes into alignment with the second sensor with respect to the axis, and wherein upon the first screw reaching the proximal travel limit during its travel along axis of the housing, the magnet comes into alignment with the third sensor with respect to the axis.

Optionally, the first screw may define a cavity axially extending therethrough, which is adapted for insertion and securing of the outer tube of the interventional catheter therein and for movable passage of an inner tube of the interventional catheter therethrough.

Optionally, the interventional handle may include an inner tube control module including a second screw extending along an axis of the housing and a second knob in rotatable, threaded engagement with the second screw, wherein the housing restricts the second knob in position with respect to the axis of the housing and restricts circumferential rotation of the second screw; and
the second screw is adapted for connection with an inner tube of an interventional catheter or with a steering wire, thereby allowing rotation of the second knob to cause movement of the inner tube or the steering wire along the axis of the housing.

Optionally, the housing may be provided with an observation window marked with graduations, each corresponding to an amount of rotation of the second knob.

To the above end, the present invention also provides an interventional delivery system, which includes the interventional handle as defined above and an interventional catheter. The interventional catheter includes an outer tube adapted to be driven by the electric or manual drive module to move along the axis of the housing.

To the above end, the present invention also provides another interventional handle, which includes a manual drive module and a conversion module.

The manual drive module includes a clutch assembly and a drive member. The clutch assembly is movable between an engaged configuration and a disengaged configuration.

The conversion module is adapted for connection with an outer tube of an interventional catheter and for conversion of power from the manual drive module into movement of the outer tube along a housing of the interventional handle.

The drive member is coupled to the conversion module via the clutch assembly when the clutch assembly is in the engaged configuration, or disallows power transmission between the drive member and the conversion module when the clutch assembly is in the disengaged configuration.

Optionally, the engaged configuration may be a forward-rotation configuration or a backward-rotation configuration, wherein the drive member is configured to transfer power to the conversion module when circumferentially rotating in a direction corresponding to the forward- or backward-rotation configuration, or to do nothing but idly slide when circumferentially rotating in a direction opposite to the direction corresponding to the forward- or backward-rotation configuration.

Optionally, the clutch assembly may include a diverter, a forward-rotation ratchet and a backward-rotation ratchet, and the conversion module may include a toothed wheel,
the diverter disposed so as to be circumferentially rotatable around an axis of the housing, the forward- and backward-rotation ratchets both disposed so as to be movable along the axis of the housing and both circumferentially restricted in position with respect to the drive member,
wherein when the diverter is rotated around the axis of the housing into an idle position, both the forward- and backward-rotation ratchets are separate from the toothed wheel, and the clutch assembly is in the disengaged configuration.

When the diverter is rotated about the axis of the housing into a forward-rotation position, the forward-rotation ratchet may engage the toothed wheel, allowing forward rotation of the drive member about the axis of the housing to drive the forward-rotation ratchet to cause forward rotation of the toothed wheel and allowing backward rotation of the drive member about the axis of the housing to cause the forward-rotation ratchet to slide over the toothed wheel without doing anything further to the latter.

When the diverter is rotated about the axis of the housing into a backward-rotation position, the backward-rotation ratchet may engage the toothed wheel, allowing backward rotation of the drive member about the axis of the housing to drive the backward-rotation ratchet to cause backward rotation of the toothed wheel and allowing forward rotation of the drive member about the axis of the housing to cause the backward-rotation ratchet to slide over the toothed wheel without doing anything further to the latter.

Optionally, the diverter may define a forward-rotation stop slot and a backward-rotation stop slot, both extending circumferentially, wherein each of the forward- and backward-rotation ratchets is provided thereon with a stop post; the stop post on the forward-rotation ratchet is inserted in the forward-rotation stop slot so as to be movable therein; the stop post on the backward-rotation ratchet is inserted in the backward-rotation stop slot so as to be movable therein; each of the forward- and backward-rotation stop slots defines an engagement recess depressed toward the toothed wheel;
when the diverter is in the forward-rotation position, the stop post on the forward-rotation ratchet is received in the engagement recess of the forward-rotation stop slot while abutting a wall thereof; and when the diverter is in the backward-rotation position, the stop post on the backward-rotation ratchet is received in the engagement recess of the backward-rotation stop slot while abutting against a wall thereof.

Optionally, each of the forward- and backward-rotation stop slots may define a disengagement recess farther away from the toothed wheel than the engagement recess thereof, wherein when the diverter is in the idle position, the stop post on the forward-rotation ratchet is received in the disengagement recess of the forward-rotation stop slot while abutting against a wall thereof, and the stop post on the backward-rotation ratchet is received in the disengagement recess of the backward-rotation stop slot while abutting against a wall thereof.

Optionally, the forward-rotation stop slot may define a forward-rotation transition slope, which extends between, and is contiguous with both, the engagement and disengagement recesses of the forward-rotation stop slot, and the backward-rotation stop slot may define a backward-rotation transition slope, which extends between, and is contiguous with both, the engagement and disengagement recesses of the backward-rotation stop slot.

Optionally, the clutch assembly may further include biasing members for biasing the forward- and backward-rotation ratchets toward the toothed wheel.

Optionally, the diverter may be coupled to the toothed wheel via point-contact features.

Optionally, the drive member may include a push/pull lever and a base, the base disposed so as to be rotatable about the axis of the housing, wherein the forward- and backward-rotation ratchets are disposed on the base so as to be movable along the axis of the housing and are restricted in position circumferentially relative to the base;
the push/pull lever is disposed on the base so as to be pivotable about a pivotal shaft between a stowed position and a deployed position, the pivotal shaft extending perpendicular to the axis of the housing; when in the stowed position, the push/pull lever does not extend beyond the opening, allowing the cover to cover and close the opening; and when in the deployed position, the push/pull lever extends radially with respect to the housing.

Optionally, the clutch assembly may further include a diverter toggle, which is provided on the diverter and inserted through the base, wherein the push/pull lever defines an avoidance notch; and when the diverter is in the idle position, the avoidance notch is aligned with the diverter toggle and allows its passage therethrough, thus allowing the push/pull lever to be pivoted into the stowed position.

Optionally, the base may be ring-shaped and disposed over an outer circumference of the diverter and define a circumferentially extending toggle slot, wherein the diverter toggle is attached to the diverter at one end and inserted through the toggle slot at the other end.

Optionally, the interventional handle may further include the housing, an electric drive module, a cover and a control module, wherein the conversion module is further adapted to convert power from the electric drive module into movement of the outer tube along the axis of the housing;
the housing defines an opening, and the cover is complementary in shape to the opening and adapted to detachably cover and close the opening; the electric drive module is disposed inside the housing, and the manual drive module is arranged within the housing in positional correspondence with the opening; and
the control module is configured to: obtain assembly information indicating whether the cover is covering and closing the opening; and if the assembly information indicates that the cover is covering and closing the opening, switch the electric drive module to an enabled mode, or if the assembly information indicates that the cover is separate from the opening, switch the electric drive module to a disabled mode.

To the above end, the present invention also provides another interventional delivery system, which includes the interventional handle as defined above and an interventional catheter. The interventional catheter includes an outer tube at least adapted to be driven by the electric drive module or the manual drive module to move along the axis of the housing.

As described above, the present invention provides an interventional handle and an interventional delivery system. The interventional handle includes a housing, an electric drive module, a manual drive module, a cover and a control module. The housing defines an opening, and the cover is complementary in shape to the opening and adapted to detachably cover and close the opening. The electric drive module is disposed inside the housing, and the manual drive module is arranged within the housing in positional correspondence with the opening. The control module is configured to: obtain assembly information indicating whether the cover is covering and closing the opening; and if the assembly information indicates that the cover is covering and closing the opening, switch the electric drive module to an enabled mode, or if the assembly information indicates that the cover is separate from the opening, switch the electric drive module to a disabled mode.

With this arrangement, when the cover is covering and closing the opening, the control module switches the electric drive module to the enabled mode, allowing electrical actuation. At the same time, the cover is covering and closing the opening, concealing the manual drive module. This optimized interventional handle layout allows for the incorporation of more other functional modules. When there is a need for manual actuation, the cover may be separated from the housing, exposing the manual drive module and thereby allowing it to be manipulated to provide manual actuation.

The present invention also provides another interventional handle and interventional delivery system. The interventional handle includes a manual drive module and a conversion module. The manual drive module includes a clutch assembly and a drive member. The clutch assembly can be moved between an engaged configuration and a disengaged configuration, and the conversion module is adapted for connection with an outer tube in an interventional catheter and for conversion of power from the manual drive module into axial movement of the outer tube along an axis of a housing of the interventional handle. When the clutch assembly is in the engaged configuration, the drive member is coupled to the conversion module via the clutch assembly. When the clutch assembly is in the disengaged configuration, power cannot be transmitted between the drive member and the conversion module.

With this arrangement, in order to address a need for manual actuation, the drive member may be coupled to the conversion module by manipulating the clutch assembly, thereby allowing for the provision of manual actuation. On the contrary, in response to a switching from manual actuation to electric actuation, power transmission between the drive member and the conversion module may be disallowed by manipulating the clutch assembly, avoiding the manual drive module from interfering with electric actuation.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 schematically illustrates an interventional handle according to an embodiment of the present invention, in which a cover is covering and closing an opening;
Fig. 2 schematically illustrates the interventional handle according to the embodiment of the present invention, in which the cover is separate from a housing;
Fig. 3 schematically illustrates the cover according to the embodiment of the present invention;
Fig. 4 shows a schematic cross-sectional view of the cover according to the embodiment of the present invention, taken along an axis thereof;
Fig. 5 shows a schematic cross-sectional view of the interventional handle according to the embodiment of the present invention, taken along an axis thereof;
Fig. 6 is an enlarged partial view showing an electric drive module and a manual drive module according to the embodiment of the present invention;
Fig. 7 shows an enlarged partial view of an inner tube control module according to the embodiment of the present invention;
Fig. 8 schematically illustrates an interventional handle according to an alternative embodiment of the present invention, in which a cover is separate from a housing;
Figs. 9a to 9c schematically illustrate how a drive member is circumferentially rotated in accordance with the alternative embodiment of the present invention;
Fig. 10 schematically illustrates a manual drive module according to the alternative embodiment of the present invention;
Fig. 11 shows a side view of the manual drive module according to the alternative embodiment of the present invention;
Fig. 12 shows a schematic cross-sectional view of the manual drive module according to the alternative embodiment of the present invention, taken along an axis thereof;
Figs. 13a and 13b schematically illustrate a forward-rotation ratchet and a backward-rotation ratchet according to the alternative embodiment of the present invention;
Fig. 14 schematically illustrates a diverter according to the alternative embodiment of the present invention;
Fig. 15a schematically illustrates the diverter according to the alternative embodiment of the present invention, which is in an idle position;
Fig. 15b schematically illustrates the diverter according to the alternative embodiment of the present invention, which is in a forward-rotation position;
Fig. 16 shows a top view of a clutch assembly according to the alternative embodiment of the present invention, which is in a disengaged configuration; and
Fig. 17 schematically illustrates the clutch assembly according to the alternative embodiment of the present invention, which is in a forward-rotation configuration.

### List of Reference Numerals

11 inner tube; 12 outer tube; 13 stability tube; 2 housing; 20 opening; 21 second magnetic attraction member; 22 engagement recess; 23 annular groove; 24 observation window; 3 electric drive module; 31 motor; 32 power transmission member; 321 first gear; 322 second gear; 33 battery; 4 manual drive module; 41 first knob; 42 clutch assembly; 421 diverter; 4211 forward-rotation stop slot; 4212 backward-rotation stop slot; 4213 forward-rotation engagement recess; 4214 backward-rotation engagement recess; 4215 forward-rotation disengagement recess; 4216 backward-rotation disengagement recess; 4217 forward-rotation transition slope; 4218 backward-rotation transition slope; 422 forward-rotation ratchet; 423 backward-rotation ratchet; 4231 backward-rotation stop post; 424 biasing member; 425 point-contact feature; 426 diverter toggle; 43 drive member; 430 base; 431 push/pull lever; 432 pivotal shaft; 433 bearing; 434 avoidance notch; 435 toggle slot; 5 cover; 51 magnet; 52 first magnetic attraction member; 53 engagement protrusion; 61 retrieve/load button; 62 release button; 63 power switch; 7 conversion module; 70 cavity; 71 first screw; 72 ball nut; 73 outer tube securing member; 74 magnet; 75 toothed wheel; 81 first sensor; 82 second sensor; 83 third sensor; 9 inner tube control module; 90 space; 91 second screw; 92 second knob; 93 inner tube securing member; 94 annular collar.

### DETAILED DESCRIPTION

Objects, advantages and features of the present invention will become more apparent upon reading the following more detailed description with reference to the accompanying drawings, which illustrate particular embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents, and the term "or" is generally employed in the sense of "and/or", "a number of" of "at least one" and "at least two" of "two or more". Additionally, the use of the terms "first", "second" and "third" herein is intended for illustration only and is not to be construed as denoting or implying relative importance or as implicitly indicating the numerical number of the referenced items. Accordingly, defining an item with "first", "second" or "third" is an explicit or implicit indication of the presence of one or at least two such items. The terms "one end" and "the other end", as well as "proximal end" and "distal end", may be used herein to generally refer to corresponding end portions, rather than precisely to the endpoints. The terms "proximal" and "distal" may be used herein to describe an interventional delivery system, which has one end intended to be inserted into the body of a patient and another end retained outside of the body for manipulation (corresponding to an interventional handle). In this sense, the term "proximal" refers to a location around the manipulation end of the delivery system out of the body, and the term "distal" refers to a location closer to the end inserted into the body and hence farther away from the manipulation end. These terms may also be used herein to describe a component being operated by an operator, such as a surgeon or clinician, manually or by hand. In this case, the term "proximal" refers to a location of the component closer to the operator, and the term "distal" refers to a location of the component closer to an interventional delivery system and hence farther away from the operator. Further, as used herein, the terms "mounting", "coupling", "connecting", "disposing" and any variants thereof should be interpreted in a broad sense. When an element is referred to as being "disposed" on another element, this is generally intended to only mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

It is an object of the present invention to provide interventional handles and interventional delivery systems, which overcome the problems with existing delivery systems arising from a suboptimal overall interventional handle layout due to an indispensable manual module.

These are described below with reference to the accompanying drawings.

Embodiments of the present invention provide an interventional delivery system, one possible use of which is to deliver a prosthetic valve to a target implantation site in the heart of a patient (e.g., at the native valvular annulus). The delivery system includes an interventional catheter, and the prosthetic valve can be loaded in the interventional catheter and hence movable forth and back therewith.

In an exemplary embodiment, the interventional catheter includes an inner tube 11 and an outer tube 12. The outer tube 12 is movably disposed over the inner tube 11, with a gap being present between their distal end portions, the prosthetic valve can be received in the gap. Thus, when the inner tube 11 and the outer tube 12 are axially advanced and retracted together within a blood vessel of a patient, the prosthetic valve carried therebetween will move in the same way. After being advanced to a location in the vicinity of the implantation site, the outer tube 12 may be proximally retracted relative to the inner tube 11, exposing the prosthetic valve (or part of the prosthetic valve). A stent of the prosthetic valve, optionally of the self-expanding type, will responsively expand into close contact with the native annulus at the implantation site, completing the implantation process. Retrieval can be achieved following a reverse process, and detailed description thereof is omitted herein. Thus, as would be appreciated, the interventional catheter operates to load, release or retrieve the prosthetic valve by creating a positional difference between the two tubes through displacing the outer tube 12 forward or backward relative to the inner tube 11.

In correspondence with the interventional catheter as discussed above, the delivery system further includes an interventional handle connected to a proximal end of the interventional catheter. The interventional handle is retained outside of a patient's body and can be manipulated by an operator to actuate the inner tube 11 and the outer tube 12. In an exemplary embodiment, the inner tube 11 may be fixedly attached to the interventional handle. Accordingly, the loading, release and retrieval of the prosthetic valve can be achieved simply by axially advancing or retracting the outer tube 12 through manipulating the interventional handle.

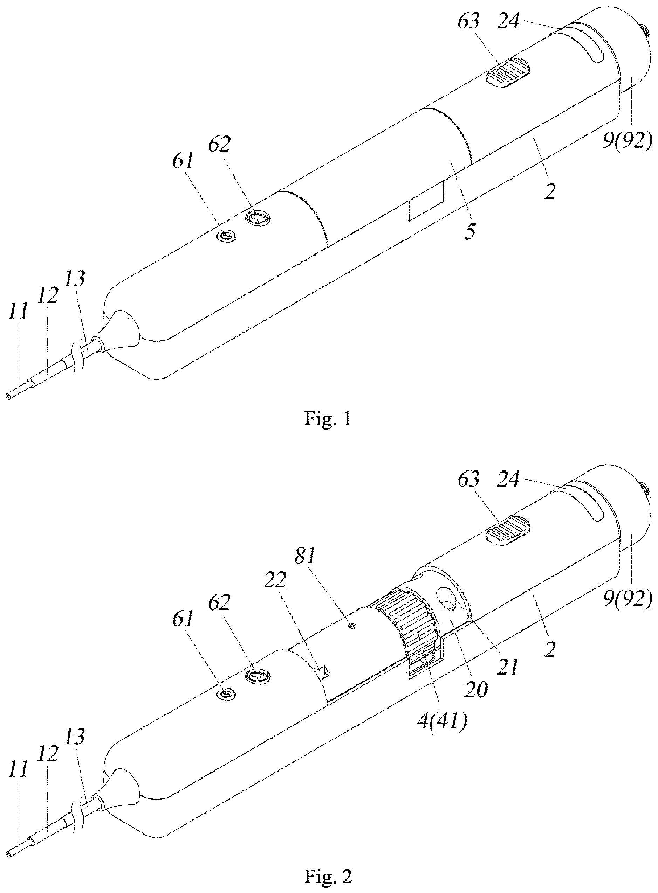

Referring to Figs. 1 to 6, an interventional handle according to an embodiment of the present invention includes a manual drive module 4 for driving the outer tube 12 to move forth and back along an axis of a housing 2. Preferably, the interventional handle further includes the housing 2, an electric drive module 3, a cover 5 and a control module (not shown). The housing 2 defines an opening 20, and the cover 5 is complementary in shape to the opening 20 and adapted to detachably cover and close the opening 20. The electric drive module 3 is disposed within the housing 2, and the manual drive module 4 is arranged in the housing 2 in positional correspondence with the opening 20. The control module is configured to obtain assembly information indicating whether the cover 5 is covering and closing the opening 20. If the assembly information indicates that the cover 5 is covering and closing the opening 20, the control module switches the electric drive module 3 to an enabled mode. Otherwise, if the information indicates that the cover 5 is separate from the opening 20, the control module switches the electric drive module 3 to a disabled mode. The electric drive module 3 and the manual drive module 4 are adapted to drive the outer tube 12 to move forth and back along the axis of the housing 2.

It should be noted that the electric drive module 3 is able to output power when in the enabled mode, and the electric drive module 3 is deactivated or disabled and cannot output power when in the disabled mode. Optionally, the electric drive module 3 may remain coupled to the outer tube 12, whether it is enabled or not. Under the control of the control module, it may be enabled to output power, or not, by means of electrical switching. Here, the electrical switching may be accomplished, for example, by an enable control signal (e.g., an electrical level), which is transmitted to the electric drive module 3 to disable or enable the electric drive module 3, or by cutting off or enabling power supply to the electric drive module 3. Those skilled in the art may properly configure this based on knowledge in the art. For example, the assembly information may be in the form of an electrical signal, such as an electrical level. After obtaining the assembly information, the control module may accordingly switch the mode of the electric drive module 3.

As shown in Figs. 2 to 4, in one exemplary embodiment, the opening 20 of the housing 2 spans roughly one-half the circumference of the housing 2, and is open to one side of the housing 2. Correspondingly, the cover 5 is generally shaped like a saddle and can snuggly fit into the opening 20 so as to be substantially flush with the surrounding housing 2, thereby covering and concealing the manual drive module 4 beneath the opening 20. Additionally, press buttons for electrically controlling the electric drive module 3 may be provided on the housing 2, including a retrieve/load button 61 and a release button 62. The retrieve/load button 61 and the release button 62 both communicatively connected to the control module. Once the retrieve/load button 61 is pressed down, an advance signal may be transmitted to the control module, and the control module may control, in response to receipt of the advance signal, the electric drive module 3 to distally move (i.e., to advance) the outer tube 12. When the release button 62 is pressed down, a retract signal may be transmitted to the control module, and the control module may control, upon receiving the retract signal, the electric drive module 3 to proximally move (i.e., to retract) the outer tube 12. Optionally, a power switch 63 may be further provided on the housing 2, which is adapted to enable or cut off power supply to the control module and the electric drive module 3. It will be understood that the housing 2, the opening 20 and the cover 5 shown in Figs. 2 to 4 are only non-limiting examples, and the housing 2, the opening 20 and the cover 5 skilled in the art may adapt their shapes to practical applications.

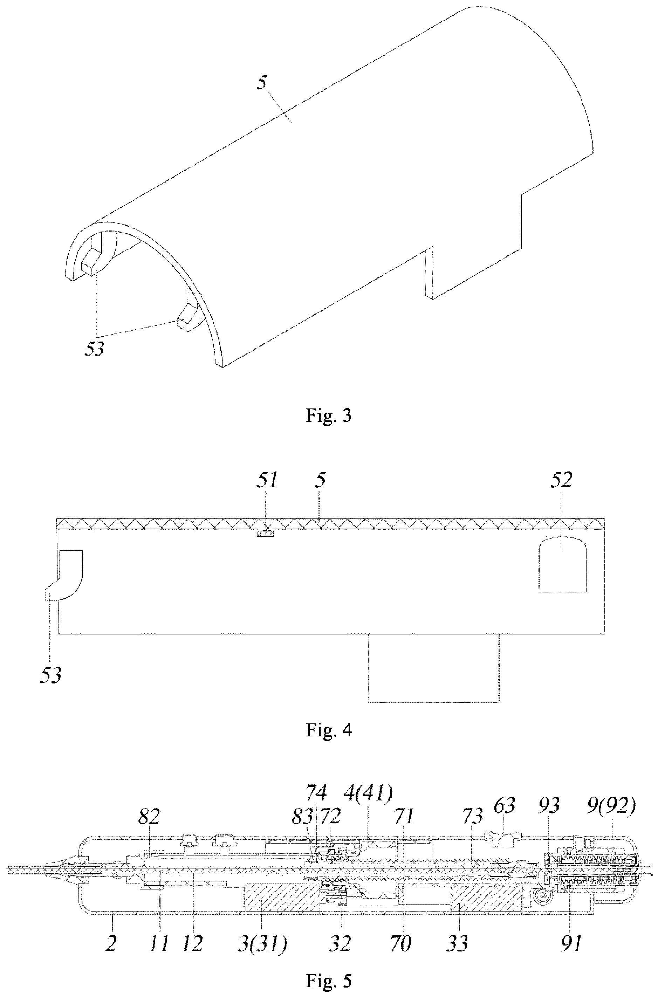

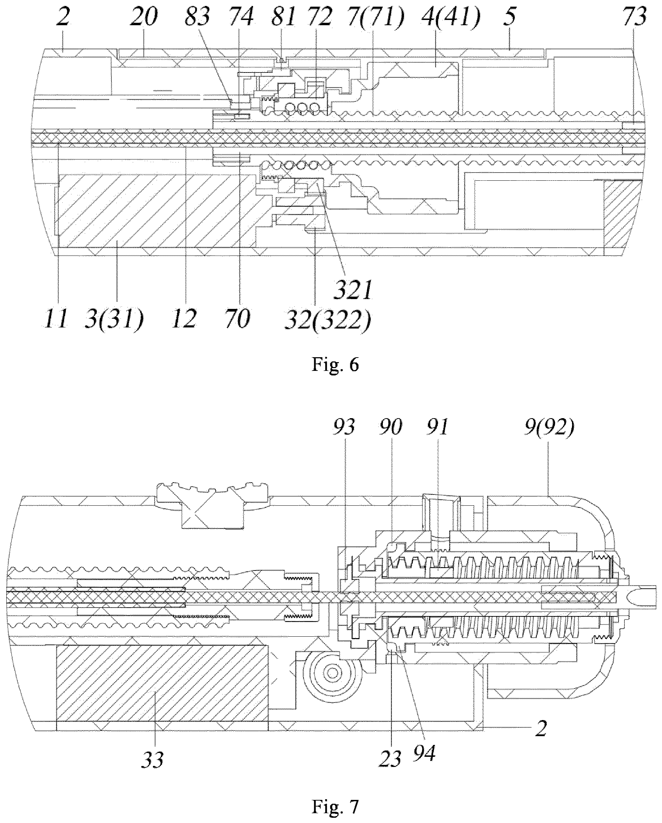

As shown in Figs. 5 and 6, the interventional handle may optionally include a conversion module 7, the conversion module 7 is adapted for connection with the outer tube 12 in the interventional catheter and convert power from the manual drive module 4 into movement of the outer tube 12 along the axis of the housing 2. Preferably, the conversion module 7 is also adapted to convert power from the electric drive module 3 into movement of the outer tube 12 along the axis of the housing 2. In an exemplary embodiment, the conversion module 7 includes a first screw 71 extending along the axis of the housing 2 and a ball nut 72 in rotatable, threaded engagement with the first screw 71. The first screw 71 is adapted for connection with the outer tube 12 in the interventional catheter. The housing 2 restricts the ball nut 72 in position with respect to the axis of the housing 2 and restricts circumferential rotation of the first screw 71. The ball nut 72 is connected to both the electric drive module 3 and the manual drive module 4. The ball nut 72 is adapted to be rotated by the electric drive module 3 or the manual drive module 4 around the first screw 71 to cause the first screw 71 to translate along the axis of the housing 2.

Optionally, the housing 2 may define a rotation-restricting guide channel (not shown) extending along its axis. The first screw 71 can move only forward or backward along the rotation-restricting guide channel while being restricted thereby from circumferential rotation. Meanwhile, restricted by the housing 2, the ball nut 72 can only rotate circumferentially, but cannot move axially.

Engagement of the first screw 71 and the ball nut 72 may be accomplished by a circular tread containing balls. When the ball nut 72 is rotated around the first screw 71, the balls can be recirculated in the ball nut 72, and the first screw 71 can be translated along the axis of the housing 2. For more structural details, reference is made to literature for conventional ball screws, and further description thereof is omitted herein. Since the ball nut 72 is connected to both the electric drive module 3 and the manual drive module 4, both the electric drive module 3 and the manual drive module 4 are coupled to the ball nut 72, both the electric drive module 3 and the manual drive module 4 are able to output driving power to the ball nut 72.

In one exemplary embodiment, the manual drive module 4 includes a first knob 41 connected coaxially to the ball nut 72. For example, the first knob 41 may be fixedly attached to the ball nut 72, or integrally formed therewith. The first knob 41 may be provided with anti-slip teeth on its outer circumference. An operator may rotate the first knob 41 around the first screw 71, thereby translating the first screw 71 forward or backward.

Optionally, the first screw 71 may define a cavity 70 axially extending therethrough, the cavity 70 is adapted for insertion and securing of the outer tube 12 of the interventional catheter therein, the cavity 70 is adapted for movable passage of the inner tube 11 of the interventional catheter therethrough. Optionally, the interventional handle may include an outer tube securing member 73, the outer tube securing member 73 is in the form of a ring defining an inner bore extending therethrough along the axis of the housing 2 and allowing the inner tube 11 therethrough. The outer tube securing member 73 is disposed in the cavity 70 and fixedly attached to the first screw 71, and the outer tube 12 is inserted into the cavity 70 from a distal end thereof and fixedly attached to the outer tube securing member 73. The inner tube 11 is passed into the cavity 70 from its distal end, through the inner bore of the outer tube securing member 73, into the outer tube 12 and to a more proximal location. With this arrangement, the first screw 71 is fixed relative to the outer tube 12, allowing the outer tube 12 to be advanced or retracted simply by rotating the first knob 41.

Optionally, the electric drive module 3 may include a motor 31 and a power transmission member 32. The motor 31 is connected to the ball nut 72 via the power transmission member 32. The motor 31 is adapt to rotate to output power under the control of the control module. In an exemplary embodiment, the power transmission member 32 includes a first gear 321 fixedly attached to the ball nut 72 and a second gear 322 fixedly attached to an output shaft of the motor 31. The first gear 321 intermeshes with the second gear 322, enabling power transmission therebetween. Of course, it will be understood that, in some other embodiments, the power transmission member 32 is not limited to being implemented as the gears, and other power transmission mechanisms commonly used in the art are also possible, such as belt and friction drives. When the electric drive module 3 is in the enabled mode, an operator can cause the motor 31 to rotate in either direction by pressing the corresponding press button, thus causing the outer tube 12 to translate forward or backward. It will be understood that since the electric drive module 3 and the manual drive module 4 are both coupled to the ball nut 72, the first knob 41 will rotate with the motor 31, and vice versa. Optionally, the interventional handle may further include a battery 33 as a power source for the motor 31 and the control module.

Since electric actuation dominates normal, ordinary control scenarios, in this embodiment, the manual drive module 4 is covered and concealed beneath the cover 5. When there is a need for a switching to the manual actuation, the cover 5 may be detached, exposing the manual drive module 4. Further, in the manual drive module, in order to prevent an undesired electric actuation action being initiated due to an inadvertent touching of any press button, which may conflict with a desired ongoing manual actuation action and thereby cause harm, the control module is configured so that when the cover 5 is separate from the opening 20, the electric drive module 3 is switched to the disabled mode. That is, once the cover 5 is detached, the electric drive module 3 will not output power, and both the retrieve/load button 61 and the release button 62 are inactive. Accordingly, even if an operator presses the retrieve/load button 61 or the release button 62, the electric drive module 3 will not respond to output power.

In order to detect whether the cover 5 is separate from the opening 20, the interventional handle may optionally include a first sensor 81, the first sensor 81 is disposed on the housing 2 in correspondence with the opening 20 and connected to the control module. The first sensor 81 is adapted to obtain assembly information about whether the cover 5 is covering and closing the opening 20. Optionally, the first sensor 81 may be a magnetic sensor, and a magnet 51 may be provided on the cover 5 in positional correspondence with the magnetic sensor. When the cover 5 is covering and closing the opening 20, the magnet 51 may be close to the first sensor 81 so that the first sensor 81 is capable of sensing a magnetic field created by the magnet 51. When this happens, it may output to the control module a piece of assembly information indicating that the cover 5 is covering and closing the opening 20. In response, the control module switches the electric drive module 3 to the enabled mode, in which when an operator presses the retrieve/load button 61 or the release button 62, the electric drive module 3 will respond to output power in corresponding direction. On the contrary, when the cover 5 is separate from the opening 20, the first sensor 81 will not sense the magnetic field from the magnet 51 and responsively output a piece of assembly information indicative of the separate cover 5 to the control module, instructing the latter to switch the electric drive module 3 to the disabled mode.

With continued reference to Fig. 5, the interventional handle may optionally include a second sensor 82 and a third sensor 83. The second sensor 82 is adapted to obtain information about a distal travel limit for the first screw 71, and the third sensor 83 is adapted to obtain information about a proximal travel limit for the first screw 71. Based on the information about the distal travel limit, the control module may prevent the electric drive module 3 from causing further distal movement of the first screw 71. Likewise, based on the information about the proximal travel limit, the control module may prevent the electric drive module 3 from causing further proximal movement of the first screw 71. The presence of the second sensor 82 and the third sensor 83 enables detection of whether travel of the first screw 71 has reached a limit. When this takes place, corresponding information may be transmitted to the control module, instructing it to prevent the electric drive module 3 from causing further movement in the corresponding direction.

In one exemplary embodiment, the second sensor 82 and the third sensor 83 are magnetic sensors, and a magnet 74 is provided on the first screw 71. The second sensor 82 is located at a distal end of an axial travel path for the first screw 71, and the third sensor 83 is located at a proximal end of the axial travel path for the first screw 71. When the first screw 71 reaches the distal limit during its travel along the axis of the housing 2, the magnet 74 will come into alignment with the second sensor 82 with respect to the axis. Similarly, when the first screw 71 reaches the proximal limit during its travel along the axis of the housing 2, the magnet 74 will come into alignment with the second third sensor 83 with respect to the axis. When the magnet 74 is aligned with the second sensor 82 or the third sensor 83 with respect to the axis, the second sensor 82 or the third sensor 83 is able to sense a magnetic field created by the magnet 74, and information is output to the control module, which indicates that the corresponding travel limit is reached. Upon receipt of information from the second sensor 82, which indicates that the distal travel limit is reached, the control module prevents the electric drive module 3 from causing further distal movement of the first screw 71. As a result, when the retrieve/load button 61 is pressed down, the electric drive module 3 will not respond at all. At this time, however, if the release button 62 is pressed down, the electric drive module 3 will still respond to drive the first screw 71 to move proximally. Likewise, when receiving information from the third sensor 83, which indicates that the proximal travel limit is reached, the control module prevents the electric drive module 3 from causing further proximal movement of the first screw 71. As a result, when the release button 62 is pressed down, the electric drive module 3 will not respond at all. At this time, however, if the retrieve/load button 61 is pressed down, the electric drive module 3 will still respond to drive the first screw 71 to move distally.

Of course, the above embodiments of magnetic sensors are only an example of the first sensor 81, the second sensor 82, and the third sensor 83, and are not limitations on the first sensor 81, the second sensor 82, and the third sensor 83. Without departing from the scope of the present invention, one of ordinary skill in the art may also configure at least one of the first sensor 81, the second sensor 82 and the third sensor 83 as any other suitable in-situ sensor, such as a tact switch, an infrared sensor, an ultrasonic sensor or the like.

Referring to Figs. 2 and 4, optionally, the cover 5 may have a connector member, and the housing 2 may have a mating connector member. These connector members are adapted to applying a force for urging the cover 5 toward the opening 20 and assembling the cover 5 at the opening 20. Optionally, the connector members may include a first magnetic attraction member 52 and a second magnetic attraction member 21, which can attract and mate with each other. The first magnetic attraction member 52 may be disposed on the cover 5, and the second magnetic attraction member 21 may be disposed at the opening 20. When the cover 5 is covering and closing the opening 20, the cover 5 may be attractively retained at the opening 20 by a magnetic force. In some embodiments, for example, one of the first magnetic attraction member 52 and the second magnetic attraction member 21 may be a magnet, and the other may be a magnetically attractable member, which can be attracted by the magnet. The magnetically attractable member may be, for example, a ferromagnetic metal block, such as an iron, nickel or iron-nickel alloy block. In some alternative embodiments, the first magnetic attraction member 52 and the second magnetic attraction member 21 may be two magnets with opposite polarity. It will be understood that, in either case, magnetic retention can be achieved by a magnetic force. In an exemplary embodiment, a magnet is provided on the cover 5, and another magnet with opposite polarity is provided on the housing 2. When the cover 5 is covering and closing the opening 20, the two magnets may be in proximity or contact with each other to create a sufficient magnetic attractive force between them, which retains the cover 5 within the housing 2 to such an extent that some effort must be made to remove the cover. This can prevent the cover 5 from accidentally falling off due to vibration during transport. Of course, the connector members are not limited to being implemented as the first magnetic attraction member 52 and the second magnetic attraction member 21 that can attract and mate with each other, and other suitable mating pairs are possible, such as those employing snap engagement, an interference fit or threaded engagement. Those skilled in the art may make an appropriate choice in accordance with the actual circumstances.

Further, with combined reference to Figs. 2 to 4, the cover 5 may define an engagement member, and the housing 2 may define a mating engagement member. When the cover 5 is covering and closing the opening 20, the cover 5 may be engaged with the opening 20 by the engagement members. On each of the cover 5 and the opening 20, the engagement and connector members may be spaced apart from each other at two ends thereof opposing along the axis of the housing 2. The mating engagement members may include, for example, an engagement protrusion 53 and an engagement recess 22 matable with the engagement protrusion 53. The engagement protrusion 53 can engage with the engagement recess 22, thereby securely retaining the cover 5 at the opening 20. In the exemplary embodiment of Figs. 2 to 4, the engagement protrusion 53 is provided at a distal end of the cover 5, and the engagement recess 22 is provided in the housing 2. Moreover, the first magnetic attraction member 52 is provided at a proximal end of the cover 5. The presence of the engagement protrusion 53 and the first magnetic attraction member 52 at the two ends of the cover 5 that oppose each other along the axis of the housing 2 enables the cover 5 to be secured to the housing 2 at both the axially opposing ends, resulting in more reliable attachment of the cover 5 to the housing 2. Of course, it will be understood that, in alternative embodiments, the engagement protrusion 53 may be provided on the housing 2, and the engagement recess 22 in the cover 5.

Referring to Fig. 7, the interventional handle may optionally include an inner tube control module 9, the inner tube control module 9 includes a second screw 91 extending along the axis of the housing 2 and a second knob 92 in rotatable, threaded engagement with the second screw 91. The housing 2 restricts the second knob 92 in position with respect to the axis of the housing 2 and restricts circumferential rotation of the second screw 91. The second screw 91 is adapted for connection with the inner tube 11 of the interventional catheter, or with a steering wire (not shown). With this arrangement, the inner tube 11 or the steering wire can be translated along the axis of the housing 2 by rotating the second knob 92.

It will be understood that since the axial position of the second knob 92 and circumferential rotation of the second screw 91 are restricted, the second knob 92 and the second screw 91 together form a threaded drive operating based on treads, which can convert rotation of the second knob 92 by an operator into translation of the second screw 91 along the axis of the housing 2.

The inner tube control module 9 may function in different ways depending on what is connected to the second screw 91. In one embodiment, the inner tube 11 has a tubular wall containing a steering wire (not shown) therein. The steering wire is passed out of the tubular wall of the inner tube 11 at a proximal location of the inner tube 11. The inner tube 11 can be bent by proximally pulling the steering wire. After the steering wire is loosened, it will move distally, allowing the inner tube 11 to recover its original linear shape. In this way, the interventional catheter can be bent and steered in a desired direction. In order to adapt the interventional catheter to a particular curved shape of a blood vessel, an operator may rotate the second knob 92 to tighten the steering wire in the inner tube 11 to bend the interventional catheter to orient its leading (distal) end in conformity with the shape of the blood vessel. This allows the delivery system to more easily advance the interventional catheter into a patient's body, or more easily bring the interventional catheter into coaxiality or concentricity with a blood vessel, as required for delivery of the prosthetic valve to a target site. Optionally, in this case, the inner tube 11 may be fixed to the housing 2 by the inner tube securing member 93, allowing the inner tube 11 to be circumferentially rotated, by turning the interventional handle, to orient the leading end of the interventional catheter in a desired direction. Through connecting the steering wire to the second screw 91, the second knob 92 can be manipulated to desirably bend and steer of the inner tube 11. In other words, in this embodiment, the inner tube control module 9 can provide the function of steering the inner tube.

In an alternative embodiment, the inner tube 11 is connected to the second screw 91. In this case, an operator can rotate the second knob 92 to cause the inner tube 11 to axially move, enabling rapid retraction of the inner tube 11. In this process, the outer tube 12 may be arbitrarily located, for example, held stationary at a certain position. In this embodiment, the inner tube control module 9 can provide the function of rapidly retracting the inner tube.

Optionally, the second screw 91 and the second knob 92 may each have a trapezoidal thread, which provides self-locking under axial loading. In this case, if the inner tube is steerable, after an operator steers the inner tube 11 into a desired shape by turning the second knob 92, it is unnecessary for him/her to hold the second knob 92 stationary. Rather, he/she may simply release the second knob 92, and the second screw 91 will still be axially locked in position by virtue of the self-locking function provided by the trapezoidal threads. That is, the desired curved shape of the inner tube 11 will be maintained, before the operator again turns the second knob 92.

Optionally, the second knob 92 may be rotatable, but not axially displaceable, relative to the housing 2. In one exemplary embodiment, the housing 2 may define an annular groove 23, and the second knob 92 may define a mating annular collar 94. When the two mate with each other, the second knob 92 will be axially restricted in position while being rotatable circumferentially. In addition, a space 90 may be left in the annular groove 23 under the annular collar 94, and in some embodiments, the annular space 90 may be filled up with balls. In this way, rolling friction will occur between the second knob 92 and the housing 2, instead of sliding friction. Otherwise, the presence of the steering wire may lead to excessive effort required to rotate the second knob 92 to actuate the second screw 91. Where appropriate, the balls may be lubricated to further reduce friction.

Preferably, the housing 2 is provided thereon with an observation window 24, the observation window 24 is marked with graduations, each corresponding to an amount of rotation of the second knob 92. With the aid of the observation window 24 and the graduations, an operator can visually determine how much the inner tube control module 9 has been rotated and hence how much it can be additionally rotated, for example, for the purpose of steering the inner tube.

On the basis of the interventional handle as discussed above, embodiments of the present invention also provide an interventional delivery system, which includes the interventional handle and an interventional catheter. The interventional catheter includes an outer tube 12, the outer tube 12 is adapted to be driven by the electric drive module 3 or the manual drive module 4 to move along the axis of the housing 2. The interventional catheter further includes an inner tube 11 and, preferably, a stability tube 13. The stability tube 13 is connected to the housing 2 at one end and disposed over the outer tube 12 at the other end, without jacketing the entire outer tube 12. It serves to help create a stable access for an interventional procedure. The delivery system may also include other components, which may operate as known in the art, and, thereby, need not be described in further detail herein.

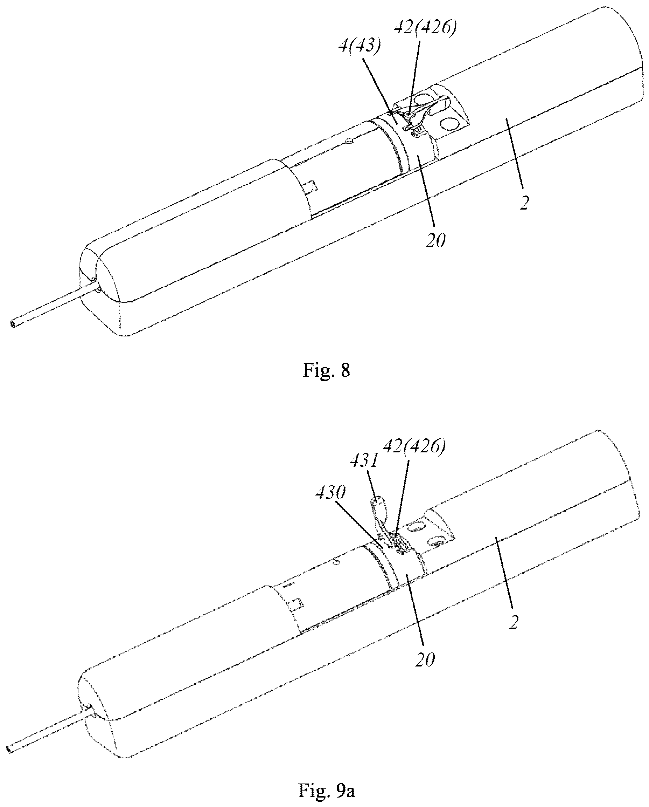

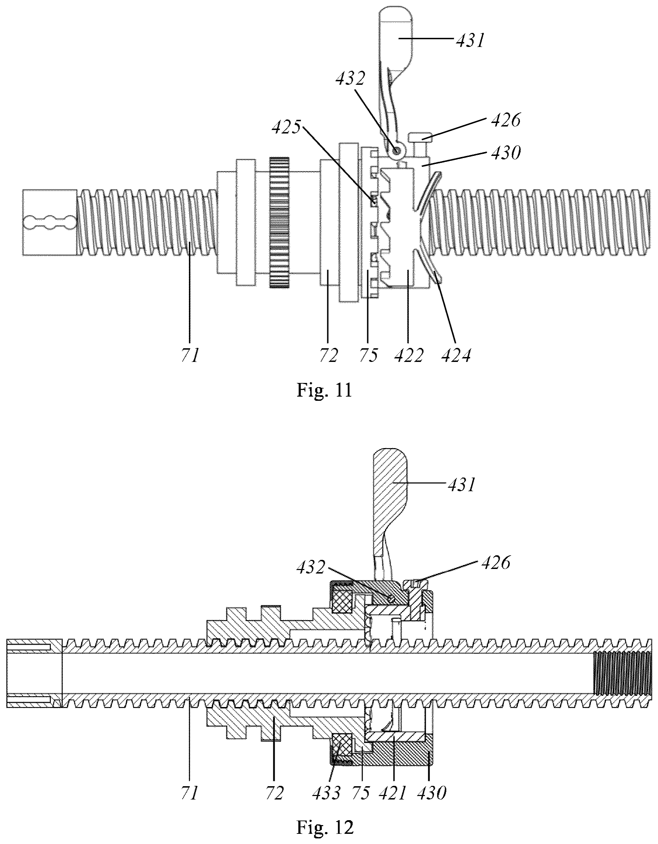

Referring to Figs. 8 to 17, in an alternative embodiment, the manual drive module 4 is structured differently from the embodiments described above. Specifically, the manual drive module 4 includes a clutch assembly 42 and a drive member 43. The clutch assembly 42 is movable between an engaged configuration and a disengaged configuration. When the clutch assembly 42 is in the engaged configuration, the drive member 43 is coupled to the conversion module 7 via the clutch assembly 42. When the clutch assembly 42 is in the disengaged configuration, power cannot be transmitted between the drive member 43 and the conversion module 7.

Thus, power transmission between the drive member 43 and the conversion module 7 can be enabled and disabled at will with the clutch assembly 42. The cover 5 may be further taken into account. Specifically, when the cover 5 is covering and closing the opening 20, the clutch assembly 42 may be moved into the disengaged configuration, disallowing power to be transmitted between the drive member 43 and the conversion module 7. At the same time, the control module may switch the electric drive module to the enabled mode, enabling electrical actuation. When there is a need for manual actuation, the cover 5 may be separated from the housing 2, exposing the manual drive module 4. Moreover, the clutch assembly 42 may be moved into the engaged configuration, coupling the drive member 43 to the conversion module 7 via the clutch assembly 42 and thereby allowing manual actuation to be provided by an operator's manipulation of the drive member 43.

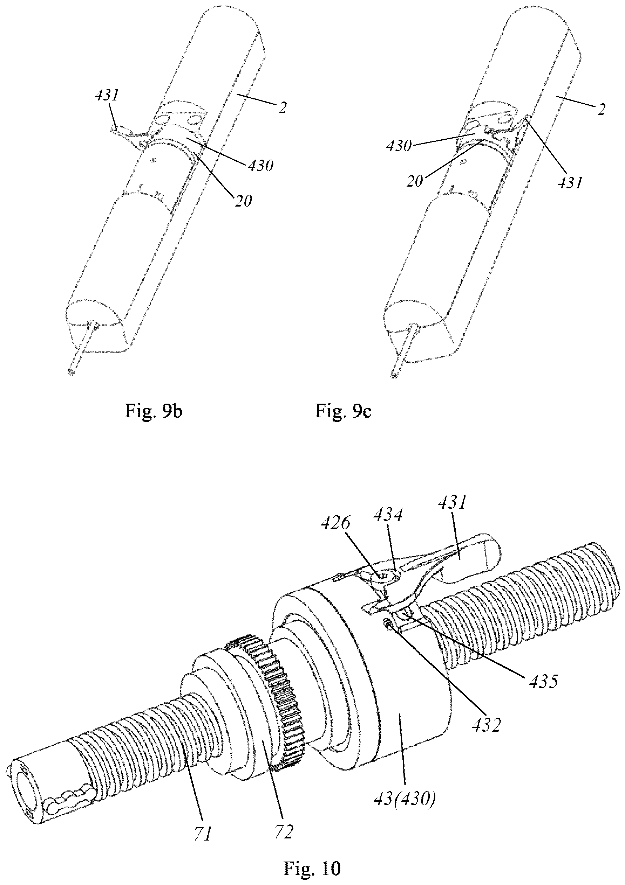

Optionally, as shown in Figs. 9a to 9c, the drive member 43 may be able to rotate circumferentially around the axis of the housing 2. Additionally, the engaged configuration may be a forward- or backward-rotation configuration. The drive member 43 may be configured to transfer power to the conversion module 7 when circumferentially rotating in a direction corresponding to the forward- or backward-rotation configuration, or to do nothing but idly slide when circumferentially rotating in a direction opposite to the direction corresponding to the forward- or backward-rotation configuration. It should be noted that, as used here, the terms "forward rotation" and "backward rotation" refer to circumferential rotation around the axis of the housing 2 in opposite directions. For example, if forward rotation is clockwise, then backward rotation will be counterclockwise. Of course, it is also possible that forward rotation is counterclockwise and backward rotation is clockwise, without limiting the scope of the present invention.

Further, when the clutch assembly 42 is in the forward-rotation configuration, the drive member 43 may remain engaged when rotating forward, but not when rotating backward. That is, when the clutch assembly 42 is in the forward-rotation configuration, the direction of rotation for the drive member 43 that corresponds to the forward-rotation configuration is forward. In this configuration, forward rotation of the drive member 43 allows power transmission to the conversion module 7 via the clutch assembly 42. At the same time, backward rotation of the drive member 43 is opposite to the direction corresponding to the forward-rotation configuration and, therefore, will not cause any power to be transferred via the clutch assembly 42. That is, the drive member 43 idly rotates in the backward direction.

Similarly, when the clutch assembly 42 is in the backward-rotation configuration, the drive member 43 may remain engaged when rotating backward, but not when rotating forward. That is, when the clutch assembly 42 is in the backward-rotation configuration, the direction of rotation for the drive member 43 that corresponds to the backward-rotation configuration is backward. In this configuration, backward rotation of the drive member 43 allows power transmission to the conversion module 7 via the clutch assembly 42. At the same time, forward rotation of the drive member 43 is opposite to the direction corresponding to the backward-rotation configuration and, therefore, will not cause any power to be transferred via the clutch assembly 42. That is, the drive member 43 idly rotates in the forward direction.

Therefore, when the clutch assembly 42 is in the forward- or backward-rotation configuration, the drive member 43 can actuate the conversion module 7 only when it rotates in one direction, and will rotate idly in the opposite direction. With this arrangement, an operator can cause the conversion module 7 to translate the outer tube 12 continuously in one direction (i.e., proximally or distally) along the axis of the housing 2 simply by repeatedly pushing and pulling the drive member 43. Thus, his/her operation and use can be facilitated.

Referring to Figs. 10 to 17, in an alternative exemplary embodiment, the clutch assembly 42 includes a diverter 421, a forward-rotation ratchet 422 and a backward-rotation ratchet 423. The conversion module 7 includes a toothed wheel 75, the toothed wheel 75 may be, for example, fixedly attached to the ball nut 72. The diverter 421 is disposed so as to be circumferentially rotatable around the axis of the housing 2, and the forward-rotation ratchet 422 and the backward-rotation ratchet 423 are both disposed so as to be movable along the axis of the housing 2. Additionally, the forward-rotation ratchet 422 and the backward-rotation ratchet 423 are restricted in position circumferentially relative to the drive member 43. When the diverter 421 is pivoted about the axis of the housing 2 to an idle position, both the forward-rotation ratchet 422 and the backward-rotation ratchet 423 are brought away from the toothed wheel 75, and the clutch assembly 42 is in the disengaged configuration. When the diverter 421 is pivoted about the axis of the housing 2 to a forward-rotation position, the forward-rotation ratchet 422 is brought into engagement with the toothed wheel 75. In this configuration, when the drive member 43 is rotated forward about the axis of the housing 2, the forward-rotation ratchet 422 will drive the toothed wheel 75 to rotate in the same direction. On the contrary, when the drive member 43 is rotated backward about the axis of the housing 2, the forward-rotation ratchet 422 will simply slide over the toothed wheel 75, without doing anything further to the forward-rotation ratchet 422. When the diverter 421 is pivoted about the axis of the housing 2 to a backward-rotation position, the backward-rotation ratchet 423 is brought into engagement with the toothed wheel 75. In this configuration, when the drive member 43 is rotated backward about the axis of the housing 2, the backward-rotation ratchet 423 will drive the toothed wheel 75 to rotate in the same direction. However, when the drive member 43 is rotated forward about the axis of the housing 2, the backward-rotation ratchet 423 will simply slide over the toothed wheel 75, without doing anything further to the backward-rotation ratchet 423.

Optionally, referring to Figs. 8 to 12, the drive member 43 may include a push/pull lever 431 and a base 430. The base 430 is disposed so as to be rotatable about the axis of the housing 2, and the forward-rotation ratchet 422 and the backward-rotation ratchet 423 are disposed on the base 430 so as to be movable along the axis of the housing 2, and the forward-rotation ratchet 422 and the backward-rotation ratchet 423 are restricted in position circumferentially relative to the base 430. The push/pull lever 431 is disposed on the base 430 so as to be pivotable about a pivotal shaft 432 between a stowed position and a deployed position. The pivotal shaft 432 extends perpendicular to the axis of the housing 2. When in the stowed position, the push/pull lever 431 does not extend beyond the opening 20, allowing the cover 5 to cover and close the opening 20. When in the deployed position, the push/pull lever 431 extends radially with respect to the housing 2.

In one exemplary embodiment, the base 430 is ring-shaped and disposed on the housing 2 so as to be rotatable about the axis of the housing 2, with a bearing 433 being arranged therebetween. The forward-rotation ratchet 422 and the backward-rotation ratchet 423 are arranged on an inner side of the base 430 and restricted in position circumferentially. With this arrangement, when circumferentially rotated, the base 430 can drive the forward-rotation ratchet 422 and the backward-rotation ratchet 423 to rotate circumferentially therewith, while the base 430 cannot restrict the positions of the forward-rotation ratchet 422 and the backward-rotation ratchet 423 along the axis of the housing 2.

Further, the push/pull lever 431 can be pivoted about the pivotal shaft 432. In order to cover and close the opening 20 with the cover 5, the push/pull lever 431 may be pivoted to the stowed position, in which it does not extend beyond the opening 20, allowing the cover 5 to cover and close the opening 20. When there is a need for manual actuation, the push/pull lever 431 may be pivoted to the deployed position, where it extends radially with respect to the housing 2 and thus can be pushed or pulled by the operator. When the push/pull lever 431 is in the deployed position, as shown in Figs. 9a to 9c, circumferentially pushing or pulling the push/pull lever 431 can circumferentially rotate the base 430 in the same direction. It will be understood that, as a result of this, the forward-rotation ratchet 422 and the backward-rotation ratchet 423 within the base 430 will circumferentially rotate in sync therewith.

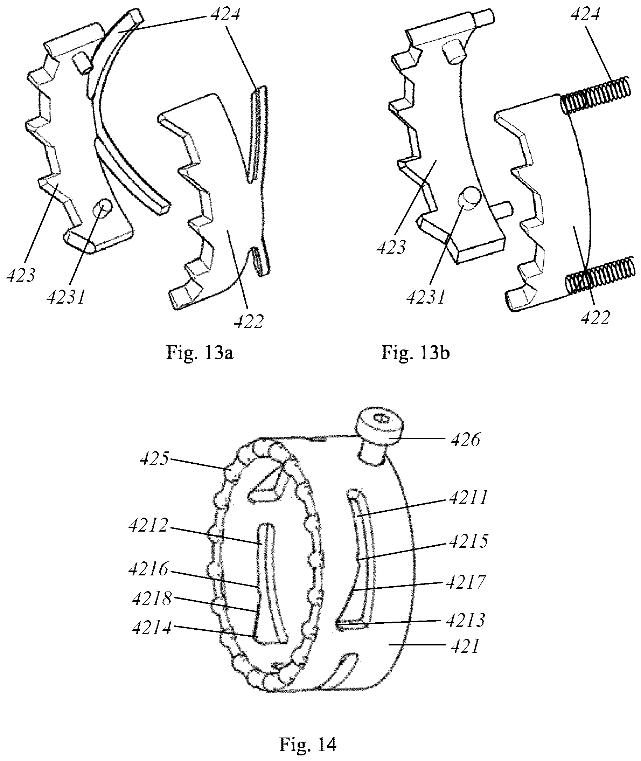

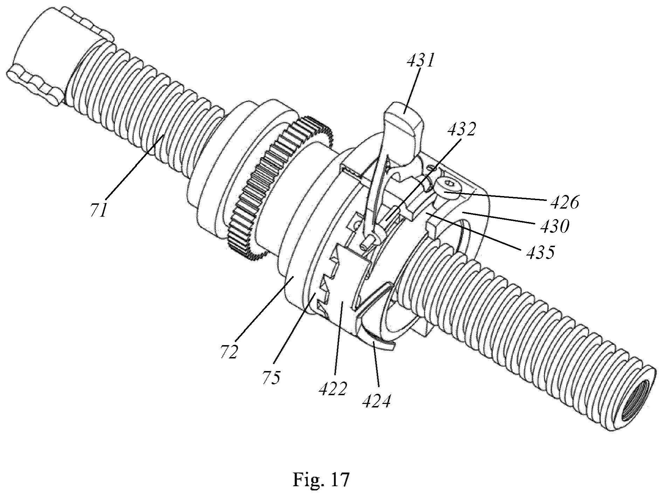

With combined reference to Figs. 13a, 13b and 17, the forward-rotation ratchet 422 and the backward-rotation ratchet 423 may be provided as a pair of ratchets with teeth oriented in opposite directions. The toothed wheel 75 may have axially raised rectangular teeth. Only the forward-rotation ratchet 422 will be described below, and the following description equally applies to the backward-rotation ratchet 423. The forward-rotation ratchet 422 can be moved toward the toothed wheel 75 along the axis of the housing 2 so that its ratchet teeth enter grooves between the teeth on the toothed wheel 75, resulting in engagement of the forward-rotation ratchet 422 with the toothed wheel 75. In this configuration, when the forward-rotation ratchet 422 is rotated forward (e.g., the forward-rotation ratchet 422 is rotated forward clockwise about the axis of the housing 2, as shown in Fig. 17), the ratchet teeth of the forward-rotation ratchet 422 may come into abutment with and urge the rectangular teeth on the toothed wheel 75, causing the toothed wheel 75 to rotate clockwise to provide power transmission. On the contrary, when the forward-rotation ratchet 422 is rotated backward (e.g., the forward-rotation ratchet 422 is rotated forward counterclockwise about the axis of the housing 2, as shown in Fig. 17), slanted surfaces of the ratchet teeth on the forward-rotation ratchet 422 will simply slide over the toothed wheel 75, without causing the latter to rotate counterclockwise. The backward-rotation ratchet 423 is structured and operates in a similar way to the forward-rotation ratchet 422, except that the ratchet teeth of the backward-rotation ratchet 423 are oriented oppositely to those of the forward-rotation ratchet 422.

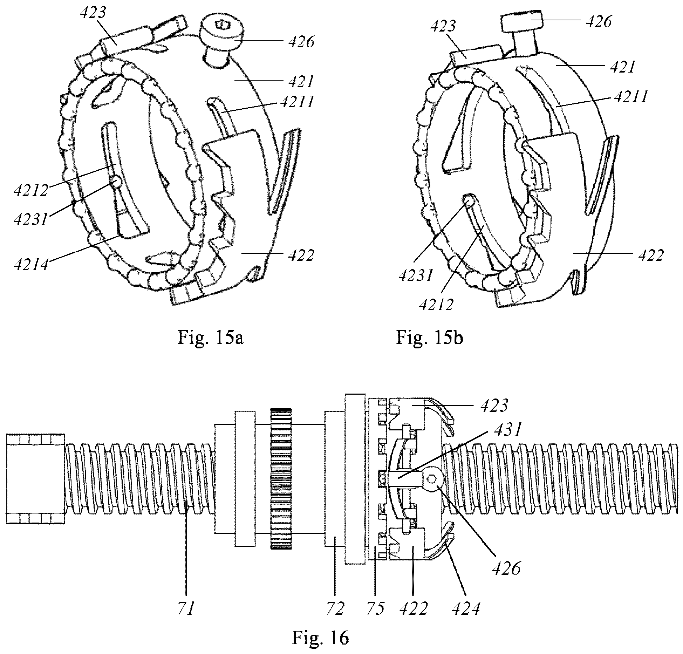

Further, the forward-rotation ratchet 422 and the backward-rotation ratchet 423 may be displaced along the axis of the housing 2 as a result of rotating the diverter 421 around the axis of the housing 2. With combined reference to Figs. 15a and 15b, the diverter 421 may be circumferentially rotated to one of three gear positions: an idle position, a forward-rotation position and a backward-rotation position. When the diverter 421 is in the idle position, both the forward-rotation ratchet 422 and the backward-rotation ratchet 423 are separate from the toothed wheel 75, and the clutch assembly 42 is in the disengaged configuration. When the diverter 421 is circumferentially rotated to the forward-rotation position, the forward-rotation ratchet 422 comes into engagement with the toothed wheel 75. When the diverter 421 is circumferentially rotated to the backward-rotation position, the backward-rotation ratchet 423 comes into engagement with the toothed wheel 75. It will be understood that displacing the forward-rotation ratchet 422 and the backward-rotation ratchet 423 along the axis of the housing 2 by circumferentially rotating the diverter 421 can be accomplished by many approaches. One of these approaches is set forth below, as an example.

In one exemplary embodiment, the diverter 421 defines a forward-rotation stop slot 4211 and a backward-rotation stop slot 4212, both extending circumferentially, and each of the forward-rotation ratchet 422 and the backward-rotation ratchet 423 is provided thereon with a stop post (for ease of description, the stop post on the forward-rotation ratchet 422 is referred to hereinafter as a "forward-rotation stop post", and the stop post on the backward-rotation ratchet 423 as a "backward-rotation stop post 4231"). The forward-rotation stop post is inserted in the forward-rotation stop slot 4211 so as to be movable therein, and the backward-rotation stop post 4231 is inserted in the backward-rotation stop slot 4212 so as to be movable therein. Each of the forward-rotation stop slot 4211 and the backward-rotation stop slot 4212 defines an engagement recess depressed toward the toothed wheel 75 (for ease of description, the engagement recess in the forward-rotation stop slot 4211 is referred to hereinafter as a "forward-rotation engagement recess 4213", and the engagement recess in the backward-rotation stop slot 4212 as a "backward-rotation engagement recess 4214"). When the diverter 421 is in the forward-rotation position, the forward-rotation stop post is received in the forward-rotation engagement recess 4213 while abutting against its wall. When the diverter 421 is in the backward-rotation position, the backward-rotation stop post 4231 is received in the backward-rotation engagement recess 4214 while abutting against its wall.

Only the forward-rotation stop slot 4211 will be described below, and the following description equally applies to the backward-rotation stop slot 4212. Since the forward-rotation engagement recess 4213 is depressed toward the toothed wheel 75, during movement of the forward-rotation stop post with circumferential rotation of the forward-rotation ratchet 422 toward the forward-rotation engagement recess 4213, the entire forward-rotation ratchet 422 will move toward the toothed wheel 75 and eventually come into engagement with the toothed wheel 75.

Optionally, each of the forward-rotation stop slot 4211 and the backward-rotation stop slot 4212 may also define a disengagement recess (for ease of description, the disengagement recess in the forward-rotation stop slot 4211 is referred to hereinafter as a "forward-rotation disengagement recess 4215", and the disengagement recess in the backward-rotation stop slot 4212 as a "backward-rotation disengagement recess 4216"). These disengagement recesses are farther away from the toothed wheel 75 than the respective engagement recesses. That is, the forward-rotation disengagement recess 4215 is farther away from the toothed wheel 75 than the forward-rotation engagement recess 4213, and the backward-rotation disengagement recess 4216 is farther away from the toothed wheel 75 than the backward-rotation engagement recess 4214. When the diverter 421 is in the idle position, the forward-rotation stop post is received in the forward-rotation disengagement recess 4215 while abutting against its wall, and the backward-rotation stop post 4231 is received in the backward-rotation disengagement recess 4216 while abutting against its wall.

Again, only the forward-rotation stop slot 4211 will be described below, and the following description equally applies to the backward-rotation stop slot 4212. Since the forward-rotation disengagement recess 4215 is depressed toward the toothed wheel 75 and located farther away from the toothed wheel 75 than the forward-rotation engagement recess 4213, when the forward-rotation stop post moves into the forward-rotation disengagement recess 4215 with circumferential rotation of the forward-rotation ratchet 422, the forward-rotation ratchet 422 is located farther away from the toothed wheel 75 than in the corresponding engagement recess and remains separate from the toothed wheel 75. The forward-rotation disengagement recess 4215, in which the forward-rotation stop post can be received while abutting against the wall of the recess, defines the idle position for rotation of the diverter 421. With this arrangement, the diverter 421 can be reliably situated in the idle position when not subject to an operator's rotating action, preventing interference of the forward-rotation ratchet 422 or the backward-rotation ratchet 423 with normal operation of the electric drive module 3.

In some embodiments, the forward-rotation ratchet 422 may be urged toward the toothed wheel 75 by the diverter 421. To this end, for example, the forward-rotation stop slot 4211 may have a width measured along the axis of the housing 2, which is equal to an outer diameter of the forward-rotation stop post. In addition, the forward-rotation stop slot 4211 may be gradually sloped along the axis of the housing 2, as it extends circumferentially. With this arrangement, as the diverter 421 is rotated circumferentially, the forward-rotation ratchet 422 will be displaced along the axis of the housing 2 under the action of the forward-rotation stop slot 4211. Since the backward-rotation stop slot 4212 is structured and operates in a similar way to the forward-rotation stop slot 4211, it need not be described in further detail herein.

Preferably, the clutch assembly 42 may further include biasing members 424, the biasing members 424 is used for biasing the forward-rotation ratchet 422 and the backward-rotation ratchet 423 toward the toothed wheel 75. In this case, the forward-rotation ratchet 422 and the backward-rotation ratchet 423 may be displaced toward the toothed wheel 75 by the biasing members 424, instead of by the diverter 421. Referring to Figs. 13a and 13b, the biasing members 424 may be implemented as resilient members, such as elastic tabs or springs, or as magnetic members each consisting of magnets with their identical poles facing each other. The present invention is not limited to any particular type of biasing members. Along the axis of the housing 2, the biasing members 424 may be attached respectively to the forward-rotation ratchet 422 and the backward-rotation ratchet 423 at one end and both to the drive member 43, for example, to the base 430, at the other end. With this arrangement, as the diverter 421 is circumferentially rotated, the forward-rotation ratchet 422 and the backward-rotation ratchet 423 will be continually biased toward the toothed wheel 75, ensuring that the forward-rotation ratchet 422 or the backward-rotation ratchet 423 can reliably engage the toothed wheel 75.

Further, the diverter 421 may be coupled to the toothed wheel 75 via point-contact features 425, the point-contact features 425 may be semispherical, pointed or spherical, for example. Preferably, the point-contact features 425 may be evenly arranged circumferentially around the diverter 421. Since there may be axial forces between the diverter 421 and the forward- and backward-rotation ratchets 422, 423, in order to ensure that the forward- and backward-rotation ratchets 422, 423 can reliably engage the toothed wheel 75, the diverter 421 is not desired to be separated from the toothed wheel 75, because this may lead to loosening that harms accuracy. In addition, axial translation of the outer tube 12 requires circumferential rotation of the ball nut 72 and the toothed wheel 75, while the diverter 421 may be maintained circumferentially stationary with respect to, or rotated by a different amount from that of, the toothed wheel 75, whether in the manual or electric actuation mode. Therefore, providing the point-contact features 425 between the diverter 421 and the toothed wheel 75 can effectively reduce friction between them.

Optionally, the forward-rotation stop slot 4211 may define a forward-rotation transition slope 4217. The forward-rotation transition slope 4217 extends between, and is contiguous with both, the forward-rotation engagement recess 4213 and the forward-rotation disengagement recess 4215. The backward-rotation stop slot 4212 may define a backward-rotation transition slope 4218, the backward-rotation transition slope 4218 extends between and is contiguous with the backward-rotation engagement recess 4214 and the backward-rotation disengagement recess 4216. The forward-rotation transition slope 4217 may be smoothly contiguous with the forward-rotation engagement recess 4213 and the forward-rotation disengagement recess 4215, and the backward-rotation transition slope 4218 may be smoothly contiguous with the backward-rotation engagement recess 4214 and the backward-rotation disengagement recess 4216. With this arrangement, circumferential rotation of the diverter 421 can cause gentle, smooth displacement of the forward-rotation ratchet 422 and the backward-rotation ratchet 423 along the axis of the housing 2.

Optionally, referring to Figs. 10 and 17, the clutch assembly 42 may further include a diverter toggle 426, the diverter toggle 426 is provided on the diverter 421 and inserted through the base 430. The push/pull lever 431 may define an avoidance notch 434. When the diverter 421 is in the idle position, the avoidance notch 434 is aligned with the diverter toggle 426 and allows the passage of the diverter toggle 426 therethrough, thus allowing the push/pull lever 431 to be pivoted into the stowed position. Optionally, the base 430 may define a circumferentially extending toggle slot 435. The diverter toggle 426 may be attached to the diverter 421 at one end and inserted through the toggle slot 435 at the other end.

In an alternative exemplary embodiment, the diverter 421 is ring-shaped, and the base 430 is disposed over an outer circumference of the diverter 421. The diverter toggle 426 radially projects from the outer circumference of the diverter 421 through the toggle slot 435 in the base 430, and thereby can be pushed and pulled by an operator. After the push/pull lever 431 is pivoted into the stowed position, the diverter toggle 426 can be received in the avoidance notch 434, avoiding either of the push/pull lever 431 and diverter toggle 426 from protruding out of the opening 20 due to interference between them. This allows the space of the opening 20 to be fully utilized, making the interventional handle compacter.

Further, the diverter toggle 426 may be aligned with the avoidance notch 434 only when the diverter 421 is in the idle position. This ensures that the clutch assembly 42 is in the disengaged configuration once the push/pull lever 431 is pivoted to the stowed position and hence that when the electric drive module 3 is activated with the opening 20 being covered and closed by the cover 5, the manual drive module 4 inside the cover 5 will not interfere with operation of the electric drive module 3.

Furthermore, inner dimensions of the avoidance notch 434 preferably match dimensions of an outer contour of the diverter toggle 426. In this way, after the diverter toggle 426 is passed through the avoidance notch 434, the push/pull lever 431 is restricted in position circumferentially and prevented the push/pull lever 431 from circumferential rotation. Thus, double security is provided to make sure that the manual drive module 4 will not interfere with operation of the electric drive module 3.

To sum up, the present invention provides an interventional handle and an interventional delivery system. The interventional handle includes a housing, an electric drive module, a manual drive module, a cover and a control module. The housing defines an opening, and the cover is complementary in shape to the opening and adapted to detachably cover and close the opening. The electric drive module is disposed within the housing, and the manual drive module is arranged in the housing in positional correspondence with the opening. The control module is configured to obtain assembly information indicating whether the cover is covering and closing the opening. If the assembly information indicates that the cover is covering and closing the opening, the control module switches the electric drive module to an enabled mode. Otherwise, if the information indicates that the cover is separate from the opening, the control module switches the electric drive module to a disabled mode. With this arrangement, when the cover is covering and closing the opening, the control module switches the electric drive module to the enabled mode, allowing electrical actuation. At the same time, the cover is covering and closing the opening, concealing the manual drive module. This optimized interventional handle layout allows for the incorporation of more other functional modules. When there is a need for manual actuation, the cover may be separated from the housing, exposing the manual drive module and thereby allowing it to be manipulated to provide manual actuation. The present invention provides another interventional handle and interventional delivery system. The interventional handle includes a manual drive module and a conversion module. The manual drive module includes a clutch assembly and a drive member. The clutch assembly can be moved between an engaged configuration and a disengaged configuration, and the conversion module is adapted for connection with an outer tube in an interventional catheter and for conversion of power from the manual drive module into axial movement of the outer tube along an axis of a housing of the interventional handle. When the clutch assembly is in the engaged configuration, the drive member is coupled to the conversion module via the clutch assembly. When the clutch assembly is in the disengaged configuration, power cannot be transmitted between the drive member and the conversion module. With this arrangement, in order to address a need for manual actuation, the drive member may be coupled to the conversion module by manipulating the clutch assembly, thereby allowing for the provision of manual actuation. On the contrary, in response to a switching from manual actuation to electric actuation, power transmission between the drive member and the conversion module may be disallowed by manipulating the clutch assembly, avoiding the manual drive module from interfering with electric actuation.

It should be noted that the foregoing embodiments may be combined in any suitable combination. The description presented above is merely that of a few preferred embodiments of the present invention and is not intended to limit the scope thereof in any sense. Any and all changes and modifications made by those of ordinary skill in the art based on the above teachings fall within the scope of the invention.

## Claims

1. An interventional handle, comprising a housing, an electric drive module, a manual drive module, a cover and a control module,
the housing provided with an opening, the cover complementary in shape to the opening and adapted to detachably cover and close the opening, the electric drive module arranged in the housing, the manual drive module arranged at the housing in positional correspondence with the opening,
the control module configured to: obtain assembly information indicating whether the cover is covering and closing the opening; and if the assembly information indicates that the cover is covering and closing the opening, switch the electric drive module to an enabled mode, or if the assembly information indicates that the cover is separate from the opening, switch the electric drive module to a disabled mode.

2. The interventional handle according to claim 1, wherein the interventional handle comprises a first sensor, the first sensor disposed at the housing in positional correspondence with the opening and connected to the control module, and the first sensor adapted to obtain the assembly information indicating whether the cover is covering and closing the opening.

3. The interventional handle according to claim 2, wherein the first sensor is a magnetic sensor, and wherein the cover is provided with a magnet in positional correspondence with the magnetic sensor.

4. The interventional handle according to claim 1, wherein the interventional handle comprises mating connector members adapted to applying a force for urging the cover toward the opening and assembling the cover at the opening.

5. The interventional handle according to claim 1, wherein the connector members include a first magnetic attraction member and a second magnetic attraction member, which can attract and mate with each other, the first magnetic attraction member disposed on the cover, the second magnetic attraction member disposed at the opening, wherein when the cover is covering and closing the opening, the cover is attractively retained at the opening by a magnetic force.

6. The interventional handle according to claim 5, wherein the cover defines an engagement member and the housing defines a mating engagement member, wherein when the cover is covering and closing the opening, the cover is engaged with the opening by the engagement members, and wherein each of the engagement members and a respective one of the connector members are spaced apart from each other along an axis of the housing at opposite ends of the cover or the opening.

7. The interventional handle according to claim 1, comprising a conversion module, the conversion module comprising a first screw arranged along an axis of the housing and a ball nut in rotatable, threaded engagement with the first screw, the first screw adapted for connection with an outer tube in an interventional catheter,
wherein the housing restricts the ball nut in position with respect to the axis of the housing and restricts circumferential rotation of the first screw, the ball nut is connected to both the electric drive module and the manual drive module, and the ball nut is adapted to be rotated around the first screw by the electric drive module or the manual drive module to drive the first screw to move along the axis of the housing.

8. The interventional handle according to claim 7, wherein the manual drive module comprises a first knob connected coaxially to the ball nut, the electric drive module comprising a motor and a power transmission member, and the motor connected to the ball nut via the power transmission member.

9. The interventional handle according to claim 7, comprising a second sensor and a third sensor, the second sensor adapted to obtain information about a distal travel limit for the first screw, the third sensor adapted to obtain information about a proximal travel limit for the first screw, wherein the control module prevents the electric drive module from causing further distal movement of the first screw based on the information about the distal travel limit and prevents the electric drive module from causing further proximal movement of the first screw based on the information about the proximal travel limit.

10. The interventional handle according to claim 9, wherein the second sensor and the third sensor are magnetic sensors, the first screw being provided with a magnet, wherein upon the first screw reaching the distal travel limit during its travel along axis of the housing, the magnet comes into alignment with the second sensor with respect to the axis, and wherein upon the first screw reaching the proximal travel limit during its travel along axis of the housing, the magnet comes into alignment with the third sensor with respect to the axis.

11. The interventional handle according to claim 7, wherein the first screw defines a cavity axially extending therethrough, the cavity adapted for insertion and securing of the outer tube of the interventional catheter therein, and the cavity adapted for movable passage of an inner tube of the interventional catheter therethrough.

12. The interventional handle according to claim 1, comprising an inner tube control module, the inner tube control module comprising a second screw extending along an axis of the housing and a second knob in rotatable, threaded engagement with the second screw, wherein the housing restricts the second knob in position with respect to the axis of the housing and restricts circumferential rotation of the second screw; and
the second screw is adapted for connection with an inner tube of an interventional catheter or with a steering wire, thereby allowing rotation of the second knob to cause movement of the inner tube or the steering wire along the axis of the housing.

13. The interventional handle according to claim 12, wherein the housing is provided with an observation window, the observation window marked with graduations, each corresponding to an amount of rotation of the second knob.

14. An interventional delivery system, comprising the interventional handle according to any one of claims 1 to 13 and an interventional catheter, the interventional catheter comprising an outer tube adapted to be driven by the electric drive module or the manual drive module to move along the axis of the housing.

15. An interventional handle, comprising a manual drive module and a conversion module,
the manual drive module comprising a clutch assembly and a drive member, the clutch assembly movable between an engaged configuration and a disengaged configuration,
the conversion module adapted for connection with an outer tube of an interventional catheter and for conversion of power from the manual drive module into movement of the outer tube along a housing of the interventional handle,
wherein when the clutch assembly is in the engaged configuration, the drive member is coupled to the conversion module via the clutch assembly, or wherein when the clutch assembly is in the disengaged configuration, it disallows power transmission between the drive member and the conversion module.

16. The interventional handle according to claim 15, wherein the engaged configuration is a forward-rotation configuration or a backward-rotation configuration, wherein the drive member is configured to transfer power to the conversion module when circumferentially rotating in a direction corresponding to the forward-rotation configuration or the backward-rotation configuration, or to do nothing but idly slide when circumferentially rotating in a direction opposite to the direction corresponding to the forward-rotation configuration or the backward-rotation configuration.

17. The interventional handle according to claim 15 or 16, wherein the clutch assembly comprises a diverter, a forward-rotation ratchet and a backward-rotation ratchet and the conversion module comprises a toothed wheel,
the diverter disposed so as to be circumferentially rotatable around an axis of the housing, the forward-rotation ratchet and the backward-rotation ratchet both disposed so as to be movable along the axis of the housing and both circumferentially restricted in position with respect to the drive member,
wherein when the diverter is rotated around the axis of the housing into an idle position, both the forward-rotation ratchet and the backward-rotation ratchet are separate from the toothed wheel, and the clutch assembly is in the disengaged configuration.

18. The interventional handle according to claim 17, wherein when the diverter is rotated about the axis of the housing into a forward-rotation position, the forward-rotation ratchet engages the toothed wheel, allowing forward rotation of the drive member about the axis of the housing to drive the forward-rotation ratchet to cause forward rotation of the toothed wheel and allowing backward rotation of the drive member about the axis of the housing to cause the forward-rotation ratchet to slide over the toothed wheel without doing anything further to the forward-rotation ratchet, and
when the diverter is rotated about the axis of the housing into a backward-rotation position, the backward-rotation ratchet engages the toothed wheel, allowing backward rotation of the drive member about the axis of the housing to drive the backward-rotation ratchet to cause backward rotation of the toothed wheel and allowing forward rotation of the drive member about the axis of the housing to cause the backward-rotation ratchet to slide over the toothed wheel without doing anything further to the backward-rotation ratchet.

19. The interventional handle according to claim 18, wherein the diverter defines a forward-rotation stop slot and a backward-rotation stop slot, both extending circumferentially, wherein each of the forward-rotation ratchet and the backward-rotation ratchet is provided thereon with a stop post; the stop post on the forward-rotation ratchet is inserted in the forward-rotation stop slot so as to be movable therein; the stop post on the backward-rotation ratchet is inserted in the backward-rotation stop slot so as to be movable therein; each of the forward-rotation stop slot and the backward-rotation stop slot defines an engagement recess depressed toward the toothed wheel;
when the diverter is in the forward-rotation position, the stop post on the forward-rotation ratchet is received in the engagement recess of the forward-rotation stop slot while abutting a wall thereof; and when the diverter is in the backward-rotation position, the stop post on the backward-rotation ratchet is received in the engagement recess of the backward-rotation stop slot while abutting against a wall thereof.

20. The interventional handle according to claim 19, wherein each of the forward-rotation stop slot and the backward-rotation stop slot defines a disengagement recess farther away from the toothed wheel than the engagement recess thereof, wherein when the diverter is in the idle position, the stop post on the forward-rotation ratchet is received in the disengagement recess of the forward-rotation stop slot while abutting against a wall thereof, and the stop post on the backward-rotation ratchet is received in the disengagement recess of the backward-rotation stop slot while abutting against a wall thereof.

21. The interventional handle according to claim 20, wherein the forward-rotation stop slot defines a forward-rotation transition slope, which extends between, and is contiguous with both, the engagement recess and the disengagement recess of the forward-rotation stop slot and the backward-rotation stop slot defines a backward-rotation transition slope, which extends between, and is contiguous with both, the engagement recess and the disengagement recess of the backward-rotation stop slot.

22. The interventional handle according to claim 19, wherein the clutch assembly further comprises biasing members for biasing the forward-rotation ratchet and the backward-rotation ratchet toward the toothed wheel.

23. The interventional handle according to claim 18, wherein the diverter is coupled to the toothed wheel via point-contact features.

24. The interventional handle according to claim 18, wherein the drive member comprises a push/pull lever and a base, the base disposed so as to be rotatable about the axis of the housing, wherein the forward-rotation ratchet and the backward-rotation ratchet are disposed on the base so as to be movable along the axis of the housing and are restricted in position circumferentially relative to the base;
the push/pull lever is disposed on the base so as to be pivotable about a pivotal shaft between a stowed position and a deployed position, the pivotal shaft extending perpendicular to the axis of the housing; when in the stowed position, the push/pull lever does not extend beyond the opening, allowing the cover to cover and close the opening; and when in the deployed position, the push/pull lever extends radially with respect to the housing.

25. The interventional handle according to claim 24, wherein the clutch assembly further comprises a diverter toggle, which is provided on the diverter and inserted through the base, wherein the push/pull lever defines an avoidance notch; and when the diverter is in the idle position, the avoidance notch is aligned with the diverter toggle and allows a passage of the diverter toggle therethrough, thus allowing the push/pull lever to be pivoted into the stowed position.

26. The interventional handle according to claim 25, wherein the base is ring-shaped and disposed over an outer circumference of the diverter, the base defining a toggle slot, which extends circumferentially, and wherein the diverter toggle is attached to the diverter at one end and inserted through the toggle slot at the other end.

27. The interventional handle according to claim 15, further comprising the housing, an electric drive module, a cover and a control module, wherein the conversion module is further adapted to convert power from the electric drive module into movement of the outer tube along the axis of the housing;
the housing defines an opening, and the cover is complementary in shape to the opening and adapted to detachably cover and close the opening; the electric drive module is disposed in the housing, and the manual drive module is arranged at the housing in positional correspondence with the opening; and
the control module is configured to: obtain assembly information indicating whether the cover is covering and closing the opening; and if the assembly information indicates that the cover is covering and closing the opening, switch the electric drive module to an enabled mode, or if the assembly information indicates that the cover is separate from the opening, switch the electric drive module to a disabled mode.

28. An interventional delivery system, comprising the interventional handle according to any one of claims 15 to 27, further comprising an interventional catheter, the interventional catheter comprising an outer tube, the outer tube at least adapted to be driven by the manual drive module to move along the axis of the housing.
